(19) Europäisches Patentamt
European Patent Office
Office européen des brevets

(11) **EP 2 910 975 B1**

(12) **EUROPEAN PATENT SPECIFICATION**

(45) Date of publication and mention of the grant of the patent:
**20.09.2017 Bulletin 2017/38**

(21) Application number: **13849654.2**

(22) Date of filing: **22.10.2013**

(51) Int Cl.:
***G01T 1/161*** *(2006.01)*

(86) International application number:
**PCT/JP2013/078602**

(87) International publication number:
**WO 2014/065286 (01.05.2014 Gazette 2014/18)**

(54) **ARRANGEMENT DETERMINING METHOD OF DETECTOR MODULE, GAMMA RAY DETECTOR, AND PET APPARATUS**

VERFAHREN ZUR BESTIMMUNG DER ANORDNUNG EINES DETEKTORMODULS, GAMMASTRAHLENDETEKTOR SOWIE PET-VORRICHTUNG

PROCÉDÉ DE DÉTERMINATION DE LA DISPOSITION D'UN MODULE DE DÉTECTEUR, DÉTECTEUR DE RAYONS GAMMA ET DISPOSITIF PET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2012 US 201213657533**

(43) Date of publication of application:
**26.08.2015 Bulletin 2015/35**

(73) Proprietor: **Toshiba Medical Systems Corporation**
**Otawara-shi, Tochigi-ken 324-8550 (JP)**

(72) Inventors:
• **WANG, Gin Chung**
**Vernon Hills, Illinois 60061 (US)**
• **BURR, Kent C.**
**Vernon Hills, Illinois 60061 (US)**
• **DU, Huini**
**Vernon Hills, Illinois 60061 (US)**
• **WANG, Jerry**
**Vernon Hills, Illinois 60061 (US)**

(74) Representative: **Moreland, David et al**
**Marks & Clerk LLP**
**Aurora**
**120 Bothwell Street**
**Glasgow G2 7JS (GB)**

(56) References cited:
**WO-A1-2008/102422       WO-A1-2008/102422**
**CN-A- 101 856 236       JP-A- H1 048 340**
**JP-A- 2006 084 309       US-A- 4 309 611**

**Description**

Technical Field

The embodiment relates to an arrangement determining method of a detector module, a gamma ray detector and a PET apparatus.

Background Art

A PET (Positron Emission Tomography) apparatus includes about several tens of or about one hundred detector modules having different timing properties, respectively. The detector modules are randomly arranged in the PET apparatus. For example, in the PET apparatus having a plurality of detector modules arranged to form a detector ring, the detector modules are generally arranged randomly in the detector ring.

In a TOF (Time of Flight) type of PET apparatus, this randomness usually results in a nonuniform time resolution in PET_FOV (Field of View) and reconstitution slice. When it is attempted to remove such nonuniformity, image reconstitution is further complicated. When it is not attempted to remove the nonuniformity, an image quality lowers.

CN 101 856 236 discloses a positron emission tomography (PET) method and device with application adaptability.

WO 2008/102422 discloses a nuclear medicine diagnostic device.

US 4,309,611 discloses a scanner for positron emission computed tomography.

Summary of Invention

Technical Problem

An object of the embodiment is to provide an arrangement determining method of a detector module, a gamma ray detector and a PET apparatus that can improve uniformity in performance of the PET apparatus.

Advantageous Effects of Invention

Improvement of uniformity in performance of the PET apparatus is provided.

Brief Description of Drawings

FIG. 1 illustrates an example of arrangement of forty detector modules included in a detector ring mounted on a PET apparatus according to an embodiment.

FIG. 2 illustrates a typical flow of a method of determining a detector module pair based on performance information according to the embodiment.

FIG. 3 illustrates a typical flow of a first arranging method of detector modules included in the detector ring mounted on the PET apparatus according to the embodiment.

FIG. 4A three-dimensionally illustrates an example of arrangement of 160 detector modules included in the detector ring mounted on the PET apparatus according to the embodiment.

FIG. 4B illustrates, in a planar form, the example of arrangement of the 160 detector modules included in the detector ring mounted on the PET apparatus according to the embodiment.

FIG. 5 illustrates a typical flow of a second arranging method of the detector modules included in the detector ring mounted on the PET apparatus according to the embodiment.

FIG. 6 illustrates an example of a structure of the PET apparatus according to the embodiment.

FIG. 7 illustrates an example of the structure of an improved PET apparatus that is used for measuring a time resolution of the detector module according to the embodiment.

FIG. 8 illustrates an example of a timing spectrum of the detector module according to the embodiment.

FIG. 9 illustrates an example of a structure of a measuring device for measuring the time resolution according to the embodiment.

FIG. 10 illustrates another structure example of the measuring device for measuring the time resolution according to the embodiment.

Description of Embodiments

According to an embodiment there is provided a method as claimed in claim 1.

According to another embodiment there is provided a gamma ray detector as claimed in claim 8.

The embodiment describes a device and a method improving uniformity of performance of a PET apparatus and, more specifically, describes a device and a method that improve the uniformity of the performance of the PET apparatus throughout its FOV range by arranging detector modules in a detector ring based on performance information of the detector modules.

For equalizing the time resolution of the detector module, and thereby further uniformizing the time resolutions throughout the PET apparatus, the embodiment provides a method of arranging the detector modules in the PET apparatus by using prior information such as preliminary performance characteristics and/or production performance characteristics of the detector modules. An advantage of improving an image quality by using TOF information is achieved over the FOV.

The embodiment provides a method of arranging, in a gamma ray detector, detector modules each including an array of scintillation crystals which convert light generating in response to incident gamma ray generating due to a pair annihilation event into an electric signal, and particularly a method including steps of obtaining performance information of each detector module, and determining a relative position of each detector module in the gamma ray detector.

The embodiment provides a gamma ray detector including a plurality of detector modules each including an array of scintillation crystals converting light generating in response to an incoming gamma ray generating due to a pair annihilation event into an electric signal. The detector modules are arranged in a detector ring based on performance characteristics of the detector modules.

The embodiment provides a method of measuring a time resolution of each detector module, and ranking the detector modules in the PET apparatus based on the time resolutions.

According to an embodiment, the detector modules are paired to minimize a difference between average time resolutions in each pair. Then, the paired detector modules are arranged in the PET apparatus such that their positions are the mirror of each other with respect to the center of the FOV.

According to an embodiment, the detector modules are arranged to reduce a probability of pairing an arbitrary detector of relatively low timing performance with another detector module of relatively low timing performance.

According to an embodiment, an algorithm is used for giving consideration to LOR (Line-of-Responses) from the detector modules when optimizing the uniformity in the time resolution.

According to an embodiment, the detector module is arranged to locate a target organ (e.g., heart) in a region of a relatively high time resolution.

An embodiment provides a method of uniformizing a time resolution in a PET apparatus by coupling a detector module of a relatively high time resolution to a reading electronic instrument of a relatively low time resolution.

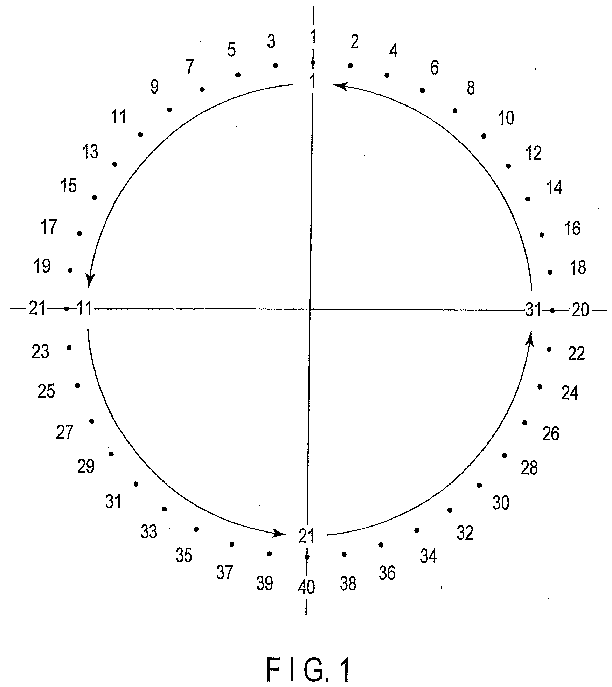

A PET apparatus according to the embodiment will be described below with reference to the drawings. FIG. 1 illustrates an example of arrangement of a plurality of detector modules in a PET apparatus. As illustrated in FIG. 1, the plurality of detector modules arranged on a circumference of a circle form a detector ring. The number of the detector modules according to the embodiment can be arbitrarily set. FIG. 1 illustrates, by way of example, the detector ring formed of the 40 detector modules. Numbers on the inner side in FIG. 1 indicate positions of the detector modules, respectively. Numbers are assigned successively and counterclockwise to the positions of the detector modules, and "1" is assigned to the top position. Numbers on the outer side in FIG. 1 indicate relative ranks of the time resolutions of the detector modules, respectively. In this example, the detector module (1st) of the highest time resolution is paired with the detector module (40th) of the lowest time resolution. Decision of forming a pair of the two detector modules is referred to as "pairing". The paired detector modules are opposed to each other. Likewise, the detector module (2nd) of the second largest time resolution is paired with the detector module (39th) of the second lowest time resolution. In this embodiment, the plurality of detector modules are arranged on the circumference of the circle such that the sum of the relative ranks of the paired two detector modules may be substantially equal to the others. The pair of two detector modules will be referred to as the "detector module pair" hereinafter.

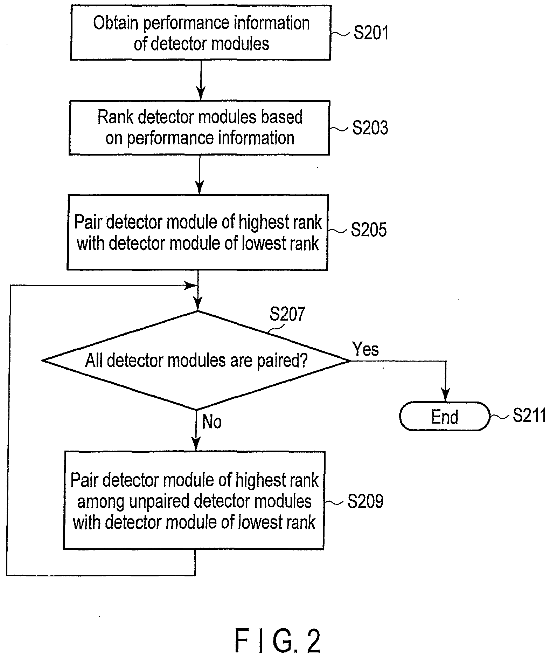

FIG. 2 illustrates a typical flow of a method of determining the detector module pairs based on the performance information of the detector modules.

In a step S201, as illustrated in FIG. 2, the performance information of the detector modules to be mounted on the detector ring is obtained. For example, the performance information is time resolution, space resolution and energy resolution of each detector module. The performance information may be other information similar to the time resolution, space resolution or energy resolution.

In a step S203, the detector modules are ranked based on the obtained performance information.

In a step S205, the detector module of the highest rank (i.e., of the highest performance) is paired with the detector module of the lowest rank.

In a step S207, it is determined whether all the detector modules are paired or not. When all the detector modules are not paired, the detector module of the highest performance rank among the unpaired detector modules is paired with the detector module of the lowest performance rank in a step S209. Then, the process returns to the step S207, and it is determined whether all the detector modules are paired or not. When it is determined in the step S207 that all the detector modules are paired, the processing of determining the detector module pairs ends in a step S211.

The processing of determining the detector module pairs is not restricted to the above processing. For example, the pairing of two detector modules may be performed to minimize differences in average time resolution between the detector module pairs.

The pairing of two detector modules may be performed, for example, to minimize a probability of pairing an arbitrary detector module of a relatively low timing performance with another detector module of a relatively low timing performance.

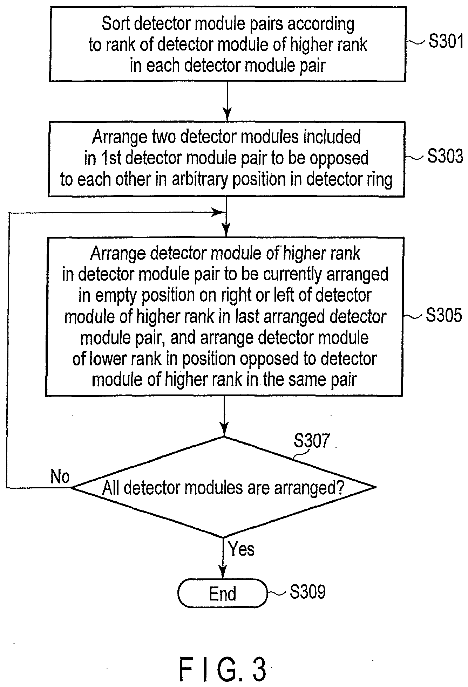

FIG. 3 illustrates a typical flow of first arranging processing of the detector module pairs in the detector ring that is mounted on the PET apparatus according to the embodiment. This first arranging processing further uniformizes the performance throughout the FOV. It is assumed that the pairing for the detector module pairs is already completed by the determining processing and others illustrated in FIG. 2 when the arranging processing in FIG. 3 starts.

In a step S301, the plurality of detector module pairs are sorted according to the rank of the detector module of the higher rank in each detector module pair. For example, the first detector module pair includes the detector module of the highest rank.

In a step S303, the two detector modules included in the first ranked detector module pair are arranged in the detector ring and are opposed to each other. The two detector modules included in the first detector module pair can be arranged at an arbitrary position in the detector ring under the restrictions that they are opposed to each other.

In a step S305, the detector module of a higher rank in the detector module pair to be currently arranged is arranged in an empty position on the right or left of the detector module in the last arranged detector module pair. The detector module of a lower rank in the detector module pair to be currently arranged is arranged in a position opposed to the detector module of the higher rank in the detector module pair in question to be currently arranged. The detector module pair to be arranged is determined as the detector module pair of the highest rank among the detector module pairs that are not yet arranged.

In a step S307, it is determined whether all the detector module pairs are arranged or not. When the detector module pair not yet arranged is present, the process returns to the step S305, and the detector module pair not yet arranged will be arranged. When it is confirmed in the step S307 that all the detector modules are arranged, the arranging processing ends in a step S309. According to this arranging method, the two detector modules belonging to the detector module pair are arranged in the PET apparatus to form mirror images with respect to the center of the FOV, respectively.

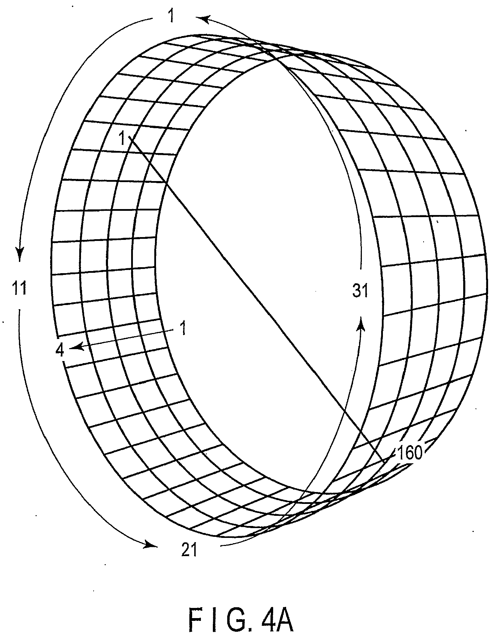

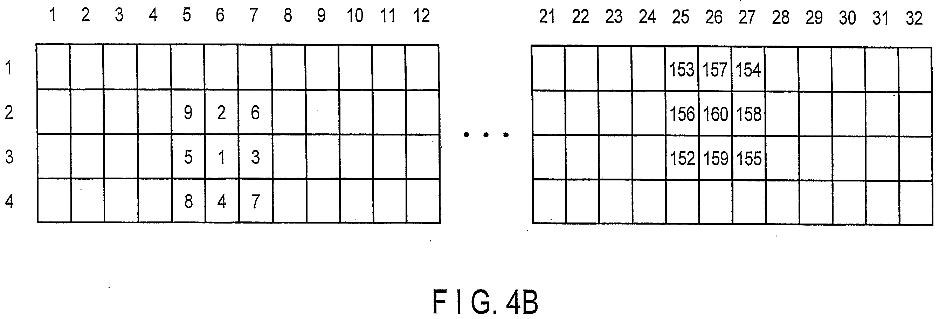

FIG. 4A three-dimensionally illustrates an example of arrangement of the 160 detector modules included in the detector ring mounted on the PET apparatus. FIG. 4B illustrates, in a planar form, the example of arrangement of the 160 detector modules included in the detector ring mounted on the PET apparatus. As illustrated in FIGS. 4A and 4B, the detector ring includes the plurality of detector modules arranged along an axial line. For example, the detector ring includes four detector module positions indicated by 1 - 4 along the axial line, respectively, and angularly shifted forty detector module positions indicated counterclockwise by 1 - 40, respectively. In FIG. 4A, a pair of "1" and "160" connected together by line represents the detector module pair formed of the 1st and 160th detector modules which are opposed to each other in the detector ring.

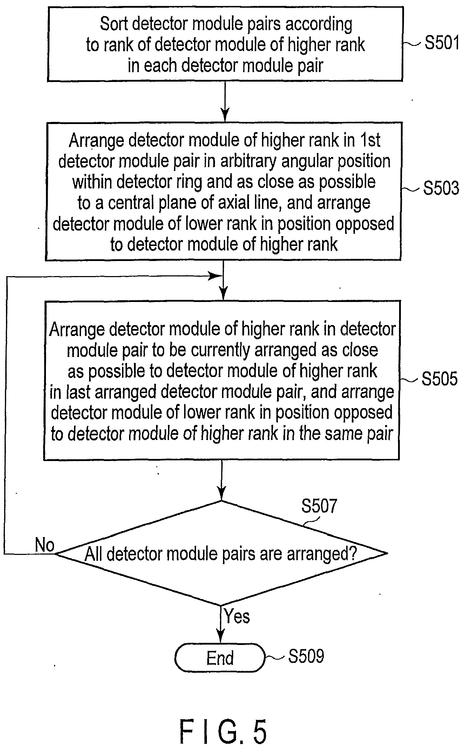

FIG. 5 illustrates a typical flow of a second arranging processing of the detector modules in the detector ring mounted on the PET apparatus according to the embodiment. This second arranging processing further uniformizes the performance throughout the FOV. Similarly to the arranging processing in FIG. 3, it is assumed that, at the start of arranging processing in FIG. 5, the pairing of the detector modules is already completed by the determining processing and others illustrated in FIG. 2.

In a step S501, the plurality of detector module pairs are sorted according to the rank of the detector module of a higher rank in each detector module pair. For example, the 1st detector module pair incudes the detector module of the highest rank.

In a step S503, the detector module of the higher rank in the 1st detector module pair is arranged in an arbitrary angular position in the detector ring and is positioned as close as possible to a central plane of the axial line. The detector module of the lower rank in the 1st detector module pair is opposed to the detector module of the higher rank.

In a step S505, the detector module of the higher rank in the detector module pair to be currently arranged is arranged as close as possible to the detector module of the higher rank in the last arranged detector module pair. The detector module of the lower rank in the detector module pair to be currently arranged is arranged in the position opposed to the module of the higher rank in the pair to be currently arranged. The detector module pair to be arranged is determined as the detector module pair of the highest rank among the detector module pairs that are not yet arranged.

In a step S507, it is determined whether all the detector module pairs are already arranged or not. When the detector module pair not yet arranged is present, the process returns to the step S505, and the detector module not yet arranged will be arranged. When it is confirmed in the step S507 that all the detector modules are arranged, the arranging processing ends in a step S509.

The processing of arranging the detector modules is not restricted to the above processing. For example, LOR from the detector modules may be taken into consideration for optimizing the uniformity of the time resolutions.

The detector modules may be arranged such that the time resolutions may be spatially different. By localizing the detector modules of relatively high performances in a specific area or positions, the time resolutions of the FOV can be spatially nonuniform. In this case, and particularly in the case of task-specific imaging (e.g., heart imaging or tumor imaging), the imaging target is arranged within a region of a relatively high time resolution. For example, in the case of the heart imaging, a patient is positioned to position the heart in a region where the time resolution is relative high.

According to the embodiment, when a reading electronic instrument has a nonuniform timing performance, the detector modules may be arranged to uniformize the time resolutions further based on a timing performance of the read-out electronics. For example, a Time-to-Digital Converter (TDC) implemented on a Field Programmable logic Array (FPGA) may have a different timing accuracy. A timing performance of the reading electronic instrument can be obtained from a past performance test conducted on each electronic board that is usually a standard portion of a manufacturing process. For uniformizing the timing performances of the PET apparatus, a detector module of a relatively high time resolution is combined with a reading electronic instrument having a relatively low timing performance so that a balanced performance may be achieved. In this case, the ranking of performance of the detector modules is determined based on the performance of a combination of the detector module and the reading electronic instrument that is assembled together with the detector module in question during manufacturing.

As described above, the detector module pair in an opposed fashion is arranged in an arbitrary position of the detector ring. This further uniformizes the quality of the LOR passing through or near the center of the detector ring.

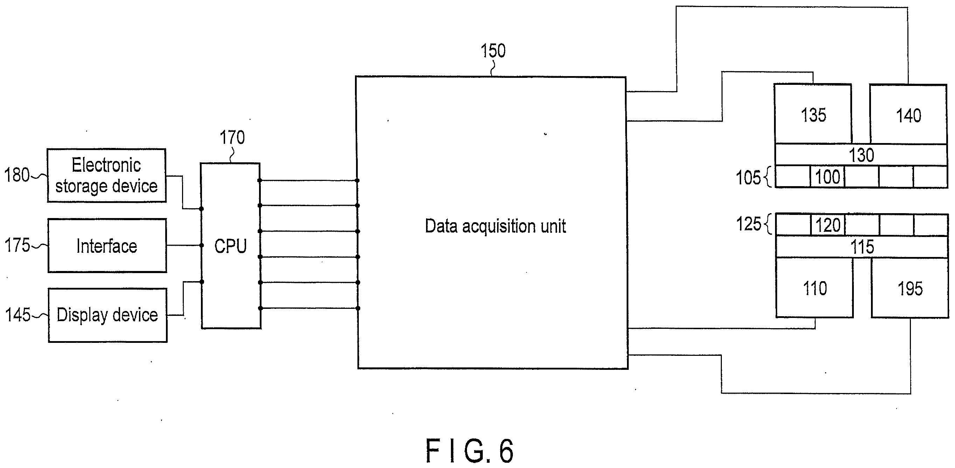

FIG. 6 illustrates an example of a structure of the PET apparatus according to the embodiment. As illustrated in FIG. 6, photomultipliers 135 and 140 are arranged on a light guide 130, and an array 105 of scintillation crystals is arranged under the light guide 130. A second array 125 of the scintillation crystals is arranged on the side opposite to the scintillation crystal 105, and a light guide 115 and photomultipliers 195 and 110 are arranged on it.

In FIG. 6, when a specimen (not illustrated) emits gamma rays, the gamma rays travel in directions mutually spaced by about 180° from each other, respectively. The scintillation crystals 100 and 120 simultaneously detect the gamma rays. When the scintillation crystals 100 and 120 simultaneously detect the gamma rays within a predetermined time, it is determined as a scintillation event. Thus, the gamma ray detector simultaneously detects the gamma rays by the scintillation crystals 100 and 120.

The photomultipliers 110, 135, 140 and 195 are connected to a data collector 150. The data collector 150 includes hardware configured to process signals provided from the photomultipliers. The data collector 150 measures an arrival time of the gamma ray. The data collector 150 produces two outputs (i.e., an output relating to a combination of the photomultipliers 135 and 140, and an output relating to a combination of the photomultipliers 110 and 195), and encodes a time of a discriminator pulse with respect to a system clock (not illustrated) with the produced two output. When the PET apparatus is of a TOF type, the data collector 150 generally produces time stamps with an accuracy of 15 - 25 ps. The data collector 150 measures the amplitudes of the signals (four signals among outputs of the data collector 150) of each photomultiplier.

The data collector 150 provides its output to a CPU 170. The CPU 170 performs signal processing on the output of the data collector 150. The signal processing includes estimation of the energy and position of the output of the data collector 150 as well as an arrival time from a time stamp output for each event. The signal processing may include application of many correction processing operations based on past calibration for improving accuracies of the estimated values of the energy, position and time. The CPU 170 may be mounted as an Application-Specific Integrated Circuit (ASIC), a Field-Programmable Gate Array (FPGA) or another Complex Programmable Logic Device (CPLD), as an individual logic gate. The mounting of the FPGA or CPLD may be encoded with VHDL, Verilog, or another arbitrary hardware description language, and the codes may be stored directly in an electronic memory in the FPGA or CPLD, or may be stored as an independent electronic memory. Further, the electronic memory may be a nonvolatile memory such as an ROM, EPROM, EEPROM, or FLASH memory. The electronic memory may also be a volatile memory such as a static or dynamic RAM, may be a processor such as a microprocessor or a microcontroller, and may be arranged for administrating mutual operations between the electronic memories or between the FPGA or CPLD and the electronic memory.

Alternatively, the CPU 170 may be mounted as a computer-readable instruction set stored in the foregoing electronic memory, hard disk drive, CD, DVD, FLASH drive, or in another arbitrary known storage medium. Further, the computer-readable instructions may be provided as, or in combination with a utility application, a background demon, or a component of an operating system, which are executed together with a processor such as an Xenon processor of the U. S. Intel corporation, an Opteron processor of the U. S. AMD corporation, or the like as well as Microsoft (registered trademark) VISTA (registered trademark), UNIX (registered trademark), Solaris (registered trademark), LINUX (registered trademark), Apple (registered trademark), MAC-OS, or another operating system already known to those skilled in the art.

When the CPU 170 performs the processing, an electronic storage device 180 stores the processed signal, or a display device 145 displays it. The electronic storage device 180 may be a hard disk drive, CD-ROM drive, DVD drive, FLASH drive, RAM, ROM, or another arbitrary electronic storage device known in the field in question. The display device 145 may be implemented as an LCD display, CRT display, plasma display, OLED, LED, or another arbitrary display device known in the field in question.

As illustrated in FIG. 6, the PET apparatus according to the embodiment includes an interface 175. The PET apparatus according to the embodiment is interface-connected to another external device and a user through the interface 175. For example, the interface 175 may be a USB interface, PCMCIA interface, Ethernet (registered trademark) interface, or another arbitrary interface known in the field in question. Also, the interface 175 may be wired or wireless, and may be a keyboard, a mouse, or another human interface device which is known in the field in question and can perform interactions with a user.

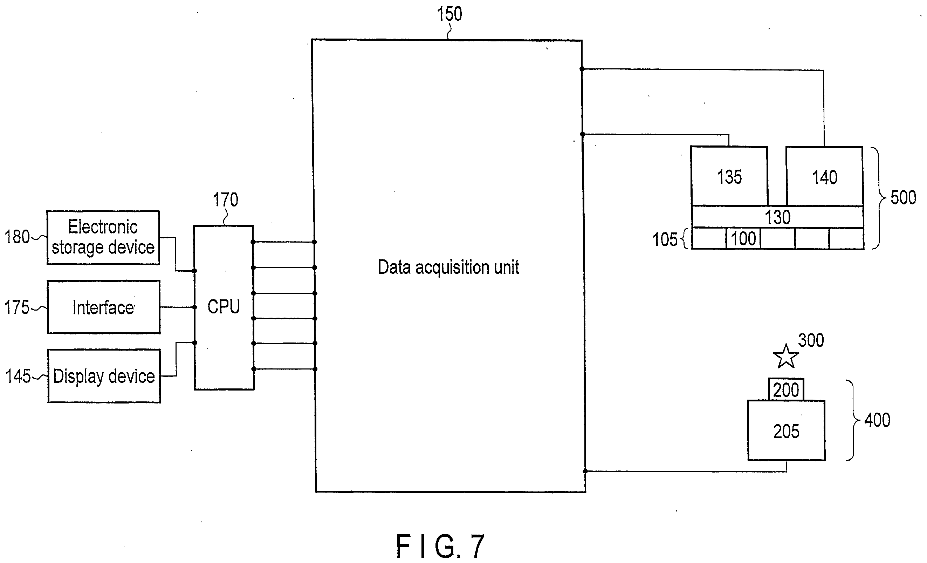

FIG. 7 illustrates an example of a structure of an improved PET apparatus used for measuring the time resolution of the detector module according to the embodiment. As illustrated in FIG. 7, a reference detector 400 and a Detector Under Test (DUT) 500 are placed on the opposite sides of radioactive isotope 300 such as $^{68}$Ge or $^{22}$Na, respectively. The radioactive isotope 300 emits a pair of gamma rays having 511 keV. The reference detector 400 includes a reference detector scintillation crystal 200 and a reference detector photosensor 205.

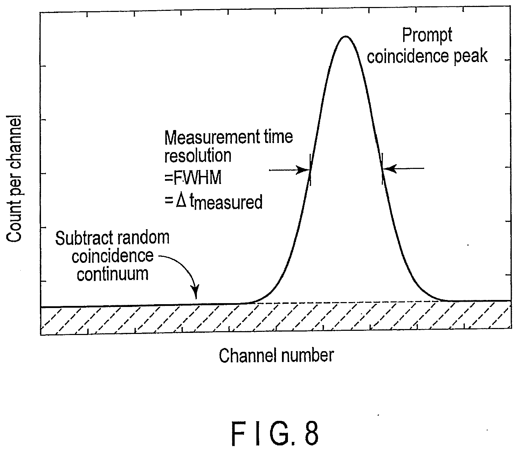

After the collection system performs pairing of events in a coincidence window, timing spectrums become visible as illustrated in FIG. 8. By an extremely small part of true coincidence events, the gamma rays detected substantially simultaneously by both the reference detector 400 and the detector 500 under test are obtained, and a prompt coincidence peak illustrated in FIG. 8 is produced. In addition to the coincidence event, each detector detects a single gamma ray of which corresponding gamma ray is not detected by the detector on the opposite side. For example, a part of the gamma rays pass through the detector without performing an interaction. Such single gamma ray may be irregularly paired by the collection system with another single gamma ray in the detector on the opposite side. Since these are not true coincidence, time distribution of them is irregular, and therefore, as illustrated in FIG. 8, random coincidence continuum of timing spectrums is obtained. Measurement time resolution $\Delta t_{measured}$ with respect to the detector pair (i.e., detector 500 under test and reference detector 400) is equal to a Full Width at Half Maximum (FWHM) of the timing spectrum after subtraction of a continuous component of the random coincidence.

From an add in quadrature of the time resolution of each detector, the measurement time resolution $\Delta t_{measured}$ is obtained. Therefore, the time resolution of the detector 500 under test is given by the following formula, assuming that the time resolution of the reference detector 400 is already known.

$$\Delta t_{measured} = \sqrt{\left(\Delta t_{reference}\right)^2 + \left(\Delta t_{DUT}\right)^2}$$

$$\Rightarrow \Delta t_{DUT} = \sqrt{\left(\Delta t_{measured}\right)^2 - \left(\Delta t_{reference}\right)^2}$$

In the formula, $\Delta t_{DUT}$ represents the time resolution of the detector 500 under test, and $\Delta t_{reference}$ represents the time resolution of the reference detector 400.

A method of measuring first the time resolution of the reference detector 400 will be described below. In this embodiment, the reference detector 400 includes a monocrystal on the photomultiplier, but alternatively it may be of a multi-pixel, in which case time resolutions of respective pixels are determined.

In the case of three reference detectors A, B and C having unknown time resolutions $\Delta t_A$, $\Delta t_B$ and $\Delta t_C$, the time resolutions of conceivable detector pairs, i.e., $\Delta t_{AB}$, $\Delta t_{AC}$ and $\Delta t_{BC}$ are measured. The unknown time resolutions may be four or more in number. Then, one set of a linear equation (in a sense of a least square because some uncertainties/noises are present during measurement) is obtained to determine $\Delta t_A$, $\Delta t_B$ and $\Delta t_C$.

$$\begin{bmatrix} 1 & 1 & 0 \\ 1 & 0 & 1 \\ 0 & 1 & 1 \end{bmatrix} \begin{bmatrix} \Delta t_A^2 \\ \Delta t_B^2 \\ \Delta t_C^2 \end{bmatrix} = \begin{bmatrix} \Delta t_{AB}^2 \\ \Delta t_{AC}^2 \\ \Delta t_{BC}^2 \end{bmatrix}$$

One of the three reference detectors A, B and C may be used as the reference detector 400. According to the embodiment, the reference detector of the lowest time resolution is used.

The alternative method of determining the time resolution of the reference detector is to construct two nominally same reference detectors (which are the same in kind and size of the scintillation crystal, in kind of the reflector around the crystal, in optically coupled compound between the crystal and the PMT, in kind of the PMT, or the like). The time resolutions of the two nominally same reference detectors can be assumed to be the same, and can be obtained by the following equation.

$$\Delta t_{measured} = \sqrt{\left(\Delta t_{reference}\right)^2 + \left(\Delta t_{reference}\right)^2} = \sqrt{2 \cdot \left(\Delta t_{reference}\right)^2} = \sqrt{2} \cdot \Delta t_{reference}$$

$$\Rightarrow \Delta t_{reference} = \frac{\Delta t_{measured}}{\sqrt{2}}$$

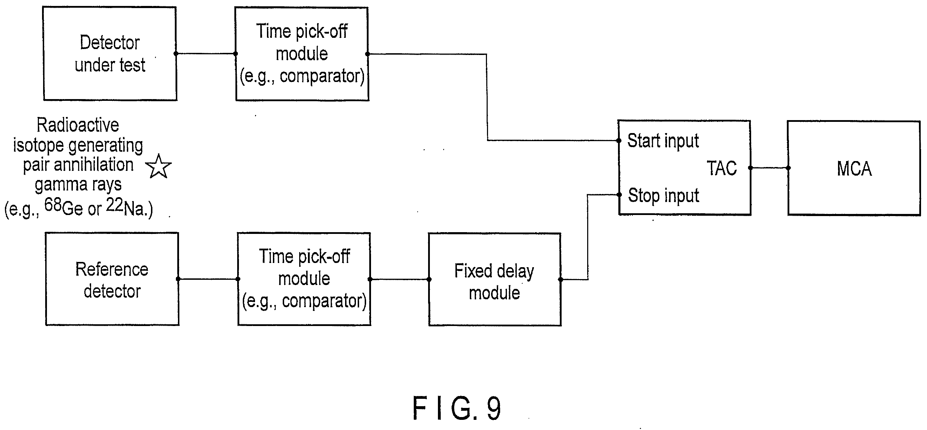

FIG. 9 illustrates a structure example of the measuring device for measuring the time resolution according to the embodiment. The reference detector and the detector under test are placed on the opposite sides of the radioactive isotope, respectively. This radioactive isotope radiates a pair of pair annihilation gamma rays having 511 keV. For example, this radioactive isotope is $^{68}$Ge, $^{22}$Na or the like. The detector under test is connected to a time pick-off module (e.g., comparator), which is connected to a start input of a TAC (Time-to-Amplitude Converter). The reference detector is connected to a time pick-off module (e.g., comparator), which is connected to a fixed delay module connected to a stop input of the TAC. The output of the TAC is connected to an MCA (Multi-Channel Analyzer). FIG. 9 illustrates only a timing channel. The measuring device according to the embodiment includes a logic circuit for recording energies of respective events and accepting only an event in a predefined energy window by the MCA.

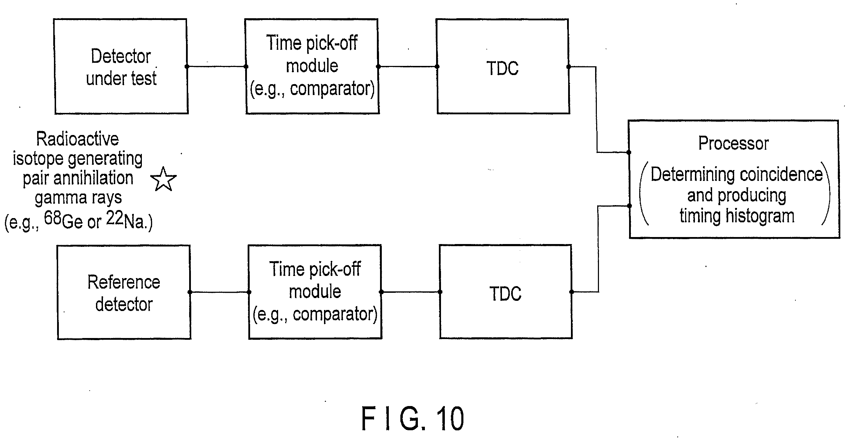

FIG. 10 illustrates another structure example of the measuring device measuring the time resolution according to the embodiment. The reference detector and the detector under test are placed on the opposite sides of the radioactive isotope, respectively. The radioactive isotope radiates a pair of pair annihilation gamma rays having 511 keV. For example, this radioactive isotope may be $^{68}$Ge, $^{22}$Na or the like. The reference detector and the detector under test are connected to time pick-off modules (e.g., comparators), which are connected to Time-to-Amplitude Converters (TDCs), respectively. The TDCs are connected to the same processing device. The processing device determines a coincidence and produces a timing histogram. FIG. 10 illustrates only a timing channel. The measuring device according to the embodiment records energies of respective events, and the processing device provides the histogram of only the event in the predefined energy window.

In the above description, it should be understood that all the processes, descriptions and blocks in the flowcharts represent parts of modules, segments or codes including one or more executable instructions for implementing specific logical functions or steps in the process. The scope of the illustrative embodiments of the invention contains alternative implementation examples, and the functions may be executed in sequences which contain the substantially simultaneous or reverse sequences according to the participating functionalities, and are different from the illustrated or discussed sequences, as can be understood by those skilled in the art.

Reference Signs List

100 ... Scintillation crystal, 105 ... Scintillation crystal array, 115 ... Light guide, 120 ... Scintillation crystal, 125 ... Scintillation crystal array, 130 ... Light guide, 135 ... Photomultiplier, 140 ... Photomultiplier, 145 ... Display device, 150 ... Data acquisition unit, 170 ... CPU, 175 ... Interface, 180 ... Electronic storage device, 195 ... Photomultiplier.

**Claims**

1. A method of determining the arrangement, in a gamma ray detector, of a plurality of detector modules (100, 120) configured to convert light generated in response to an incident gamma ray generated due to a pair annihilation event into an electric signal, comprising:

   obtaining (S201) performance information of each of the plurality of detector modules (100, 120);
   determining relative positions of the plurality of detector modules (100, 120) in the gamma ray detector based on the obtained performance information; and
   arranging the plurality of detector modules (100, 120) in the gamma ray detector based on the determined relative position to form a detector ring of the gamma ray detector;

**CHARACTERISED IN THAT**:

the step of determining the relative positions determines (S205, S209) a plurality of pairs from among the plurality of detector modules (100, 120) based on the obtained performance information; and
the arranging step arranges (S303, S305) the plurality of pairs in the detector ring such that the two detector modules forming each of the plurality of pairs are spaced by 180° from each other in the detector ring.

2. The method of determining the arrangement of the detector modules (100, 120) according to claim 1, wherein the obtaining step obtains time resolution information of each of the plurality of detector modules (100, 120) as the performance information.

3. The method of determining the arrangement of the detector modules (100, 120) according to claim 1, wherein the step of determining the relative positions determines the relative position of each of the plurality of detector modules (100, 120) to uniformize at least partially the time resolution across the gamma ray detectors.

4. The method of determining the arrangement of the detector modules (100, 120) according to claim 1, wherein each of the plurality of detector modules (100, 120) has a data acquisition unit receiving the electric signal, the method of determining the arrangement of the data detector modules (100, 120) further comprises determining performance information of each of the data acquisition units, and
the step of determining the relative positions further determines the relative positions based on the performance information of each of the acquisition units.

5. The method of determining the arrangement of the detector modules (100, 120) according to claim 1, wherein the step of determining the relative positions ranks the plurality of detector modules (100, 120) based on the performance information, and determines the plurality of pairs from among the plurality of detector modules (100, 120) such that the plurality of pairs have substantially the same rank.

6. The method of determining the arrangement of the detector modules (100, 120) according to claim 1, wherein the step of determining the relative positions determines the plurality of pairs from among the plurality of detector modules (100, 120) such that a probability of pairing the two detector modules (100, 120) with ranks lower than a predetermined rank is lower than a predetermined threshold.

7. The method of determining the arrangement of the detector modules (100, 120) according to claim 1, wherein the step of determining the relative positions further includes determining of the relative positions based on a plurality of LORs from the plurality of detector modules (100, 120) to optimize a time resolution uniformity of the gamma ray detectors.

8. A gamma ray detector comprising:

a plurality of detector modules (100, 120) arranged to form a detector ring, wherein each of the plurality of detector modules (100, 120) is configured to convert light generated in response to an incident gamma ray generated due to a pair annihilation event into an electric signal, and is arranged in the detector ring at a relative position determined based on a performance characteristic of each of the plurality of detector modules (100, 120);
**CHARACTERISED IN THAT**:

the relative positions of a plurality of pairs from among the plurality of detector modules (100, 120) is determined based on the obtained performance information; and
the plurality of pairs are arranged in the detector ring such that the two detector modules forming each of the plurality of pairs are spaced by 180° from each other in the detector ring.

9. A PET apparatus comprising a gamma ray detector according to Claim 8.

**Patentansprüche**

1. Verfahren zum Bestimmen der Anordnung mehrerer Detektormodule (100, 120), die zum Umwandeln von Licht, das ansprechend auf einen einfallenden Gammastrahl, der aufgrund eines Paarvernichtungsereignisses erzeugt wird, erzeugt wird, in ein elektrisches Signal ausgebildet sind, in einem Gammastrahlendetektor, mit:

Erhalten (S201) von Leistungsinformation jedes der mehreren Detektormodule (100, 120);
Bestimmen von Relativpositionen der mehreren Detektormodule (100, 120) in dem Gammastrahlendetektor basierend auf der erhaltenen Leistungsinformation; und
Anordnen der mehreren Detektormodule (100, 120) in dem Gammastrahlendetektor basierend auf der bestimmten Relativposition zum Ausbilden eines Detektorrings des Gammastrahlendetektors;
**dadurch gekennzeichnet, dass**:

der Schritt zum Bestimmen der Relativpositionen basierend auf der erhaltenen Leistungsinformation mehrere Paare der mehreren Detektormodule (100, 120) bestimmt (S205, S209); und
der Anordnungsschritt die mehreren Paare in dem Detektorring derart anordnet (S303, S305), dass die zwei Detektormodule, die jedes der mehreren Paare bilden, in dem Detektorring voneinander durch 180° beanstandet sind.

2. Verfahren zum Bestimmen der Anordnung der Detektormodule (100, 120) nach Anspruch 1, bei dem der Erhaltungsschritt Zeitauflösungsinformation jedes der mehreren Detektormodule (100, 120) als die Leistungsinformation erhält.

3. Verfahren zum Bestimmen der Anordnung der Detektormodule (100, 120) nach Anspruch 1, bei dem der Schritt zum Bestimmen der Relativpositionen die Relativpositionen jedes der mehreren Detektormodule (100, 120) zum zumindest teilweisen Ausgleichen der zeitlichen Auflösung der Gammastrahlendetektoren bestimmt.

4. Verfahren zum Bestimmen der Anordnung der Detektormodule (100, 120) nach Anspruch 1, bei dem jedes der mehreren Detektormodule (100, 120) eine Datenerfassungseinheit aufweist, die das elektrische Signal empfängt,
das Verfahren zum Bestimmen der Anordnung der Datendetektormodule (100, 120) ferner ein Bestimmen von Leistungsinformation der jeweiligen Datenerfassungseinheiten beinhaltet, und
der Schritt zum Bestimmen der Relativpositionen ferner die Relativpositionen basierend auf der Leistungsinformation der jeweiligen Erfassungseinheiten bestimmt.

5. Verfahren zum Bestimmen der Anordnung der Detektormodule (100, 120) nach Anspruch 1, bei dem der Schritt zum Bestimmen der Relativpositionen die mehreren Detektormodule (100, 120) basierend auf der Leistungsinformation klassifiziert und die mehreren Paare der mehreren Detektormodule (100, 120) derart bestimmt, dass die mehreren Paare im Wesentlichen dieselbe Klassifizierung aufweisen.

6. Verfahren zum Bestimmen der Anordnung der Detektormodule (100, 120) nach Anspruch 1, bei dem der Schritt zum Bestimmen der Relativpositionen die mehreren Paare der mehreren Detektormodule (100, 120) derart bestimmt, dass eine Wahrscheinlichkeit einer Paarung der zwei Detektormodule (100, 120) mit Klassifizierungen, die niedriger als eine vorbestimmte Klassifizierung sind, niedriger als eine vorbestimmte Schwelle ist.

7. Verfahren zum Bestimmen der Anordnung der Detektormodule (100, 120) nach Anspruch 1, bei dem der Schritt zum Bestimmen der Relativpositionen ferner ein Bestimmen der Relativposition basierend auf mehreren LOR der mehreren Detektormodule (100, 120) zum Optimieren einer Gleichmäßigkeit einer zeitlichen Auflösung der Gammastrahlendetektoren beinhaltet.

8. Gammastrahlendetektor mit:

mehreren Detektormodulen (100, 120), die zum Ausbilden eines Detektorrings angeordnet sind, bei dem jedes der mehreren Detektormodule (100, 120) zum Umwandeln von Licht, das ansprechend auf einen einfallenden Gammastrahl, der aufgrund eines Paarvernichtungsereignisses erzeugt wird, erzeugt wird, in ein elektrisches Signal ausgebildet ist und in dem Detektorring an einer Relativposition angeordnet ist, die basierend auf einer Leistungscharakteristik jedes der mehreren Detektormodule (100, 120) bestimmt wird;
**dadurch gekennzeichnet, dass**
die Relativpositionen mehrerer Paare der mehreren Detektormodule (100, 120) basierend auf der erhaltenen Leistungsinformation bestimmt werden; und
die mehreren Paare in dem Detektorring derart angeordnet sind, dass die zwei Detektormodule, die jedes der mehreren Paare bilden, in dem Detektorring voneinander durch 180° beabstandet sind.

**9.** PET-Vorrichtung mit einem Gammastrahlendetektor nach Anspruch 8.

**Revendications**

**1.** Procédé de détermination de l'agencement, dans un détecteur de rayons gamma, d'une pluralité de modules de détecteur (100, 120) qui sont configurés de manière à convertir une lumière qui est générée en réponse à un rayonnement gamma incident qui est généré du fait d'un événement d'annihilation de paire(s) selon un signal électrique, comprenant :

l'obtention (S201) d'une information de performance de chacun de la pluralité de modules de détecteur (100, 120) ;
la détermination de positions relatives de la pluralité de modules de détecteur (100, 120) dans le détecteur de rayons gamma sur la base de l'information de performance obtenue ; et
l'agencement de la pluralité de modules de détecteur (100, 120) dans le détecteur de rayons gamma sur la base de la position relative déterminée de manière à former un anneau de détecteurs du détecteur de rayons gamma,
**caractérisé en ce que** :

l'étape de détermination des positions relatives détermine (S205, S209) une pluralité de paires à partir de la pluralité de modules de détecteur (100, 120) sur la base de l'information de performance obtenue ; et
l'étape d'agencement agence (S303, S305) la pluralité de paires dans l'anneau de détecteurs de telle sorte que les deux modules de détecteur qui forment chacune de la pluralité de paires soient espacés de 180° l'un de l'autre dans l'anneau de détecteurs.

**2.** Procédé de détermination de l'agencement des modules de détecteur (100, 200) selon la revendication 1, dans lequel :

l'étape d'obtention obtient une information de résolution temporelle de chacun de la pluralité de modules de détecteur (100, 120) en tant qu'information de performance.

**3.** Procédé de détermination de l'agencement des modules de détecteur (100, 120) selon la revendication 1, dans lequel :

l'étape de détermination des positions relatives détermine la position relative de chacun de la pluralité de modules de détecteur (100, 120) de manière à uniformiser au moins partiellement la résolution temporelle sur les détecteurs de rayons gamma.

**4.** Procédé de détermination de l'agencement des modules de détecteur (100, 120) selon la revendication 1, dans lequel :

chacun de la pluralité de modules de détecteur (100, 120) comporte une unité d'acquisition de données qui reçoit le signal électrique ;
le procédé de détermination de l'agencement des modules de détecteur de données (100, 120) comprend en outre la détermination d'une information de performance de chacune des unités d'acquisition de données ; et
l'étape de détermination des positions relatives détermine en outre les positions relatives sur la base de l'information de performance de chacune des unités d'acquisition.

**5.** Procédé de détermination de l'agencement des modules de détecteur (100, 120) selon la revendication 1, dans lequel :

l'étape de détermination des positions relatives classe la pluralité de modules de détecteur (100, 120) sur la base de l'information de performance, et détermine la pluralité de paires à partir de la pluralité de modules de détecteur (100, 120) de telle sorte que les paires de la pluralité de paires présentent sensiblement le même rang de classement.

**6.** Procédé de détermination de l'agencement des modules de détecteur (100, 120) selon la revendication 1, dans lequel :

l'étape de détermination des positions relatives détermine la pluralité de paires à partir de la pluralité de modules de détecteur (100, 120) de telle sorte qu'une probabilité de groupage par paire des deux modules de détecteur (100, 120) dont des rangs de classement sont inférieurs à un rang de classement prédéterminé soit inférieure à un seuil prédéterminé.

**7.** Procédé de détermination de l'agencement des modules de détecteur (100, 120) selon la revendication 1, dans lequel :

l'étape de détermination des positions relatives inclut en outre la détermination des positions relatives sur la base d'une pluralité de LOR à partir de la pluralité de modules de détecteur (100, 120) de manière à optimiser une uniformité de résolution temporelle des détecteurs de rayons gamma.

**8.** Détecteur de rayons gamma comprenant :

une pluralité de modules de détecteur (100, 120) qui sont agencés de manière à former un anneau de détecteurs, dans lequel :

chacun de la pluralité de modules de détecteur (100, 120) est configuré de manière à convertir une lumière qui est générée en réponse à un rayonnement gamma incident qui est généré du fait d'un événement d'annihilation de paire(s) selon un signal électrique, et il est agencé dans l'anneau de détecteurs au niveau d'une position relative qui est déterminée sur la base d'une caractéristique de performance de chacun de la pluralité de modules de détecteur (100, 120),
**caractérisé en ce que** :

les positions relatives d'une pluralité de paires prises parmi la pluralité de modules de détecteur (100, 120) sont déterminées sur la base de l'information de performance obtenue ; et
les paires de la pluralité de paires sont agencées dans l'anneau de détecteurs de telle sorte que les deux modules de détecteur qui forment chacune de la pluralité de paires soient espacés de 180° l'un par rapport à l'autre dans l'anneau de détecteurs.

**9.** Appareil PET comprenant un détecteur de rayons gamma selon la revendication 8.

FIG. 1

Obtain performance information
of detector modules ~ S201

Rank detector modules based
on performance information ~ S203

Pair detector module of highest rank
with detector module of lowest rank ~ S205

S207

All detector modules are paired? — Yes

No

End ~ S211

Pair detector module of highest rank
among unpaired detector modules
with detector module of lowest rank ~ S209

F I G. 2

13

```
┌─────────────────────────────────────┐
│     Sort detector module pairs according      │
│  to rank of detector module of higher rank    │ ─── S301
│         in each detector module pair          │
└─────────────────────────────────────┘
                    │
                    ▼
┌─────────────────────────────────────┐
│      Arrange two detector modules included     │
│     in 1st detector module pair to be opposed  │ ─── S303
│  to each other in arbitrary position in detector ring │
└─────────────────────────────────────┘
                    │
                    ▼
┌─────────────────────────────────────┐
│      Arrange detector module of higher rank    │
│  in detector module pair to be currently arranged │
│     in empty position on right or left of detector │
│  module of higher rank in last arranged detector  │ ─── S305
│          module pair, and arrange detector module  │
│      of lower rank in position opposed to detector │
│        module of higher rank in the same pair      │
└─────────────────────────────────────┘
                    │
                    ▼
              S307
       ╱─────────────────────╲
  No  ╱  All detector modules are arranged?  ╲
 ─────╲                                       ╱
       ╲─────────────────────╱
                    │ Yes
                    ▼
           (    End    ) ─── S309
```

F I G. 3

FIG. 4A

FIG. 4B

16

Sort detector module pairs according
to rank of detector module of higher rank
in each detector module pair
~ S501

Arrange detector module of higher rank in 1st
detector module pair in arbitrary angular position
within detector ring and as close as possible
to a central plane of axial line, and arrange
detector module of lower rank in position opposed
to detector module of higher rank
~ S503

Arrange detector module of higher rank in detector
module pair to be currently arranged as close
as possible to detector module of higher rank
in last arranged detector module pair, and arrange
detector module of lower rank in position opposed
to detector module of higher rank in the same pair
~ S505

S507

No — All detector module pairs are arranged?

Yes

End ~ S509

F I G. 5

F I G. 6

F I G. 7

EP 2 910 975 B1

FIG. 8

```
┌──────────────┐     ┌──────────────────┐
│   Detector   │─────│  Time pick-off   │────────────┐
│  under test  │     │     module       │            │
│              │     │(e.g., comparator)│            │
└──────────────┘     └──────────────────┘            │
                                                     │
Radioactive                                          │          ┌──────────────┐   ┌──────────┐
isotope generating                                   └──────────│ Start input  │   │          │
pair annihilation  ☆                                            │          TAC │───│   MCA    │
gamma rays                                          ┌───────────│  Stop input  │   │          │
(e.g., 68Ge or 22Na.)                               │           └──────────────┘   └──────────┘
                                                    │
┌──────────────┐     ┌──────────────────┐     ┌──────────────┐  │
│  Reference   │─────│  Time pick-off   │─────│ Fixed delay  │──┘
│   detector   │     │     module       │     │   module     │
│              │     │(e.g., comparator)│     │              │
└──────────────┘     └──────────────────┘     └──────────────┘
```

## F I G. 9

```
┌──────────────┐      ┌──────────────────┐      ┌──────────────┐
│   Detector   │      │  Time pick-off   │      │              │
│  under test  │──────│     module       │──────│     TDC      │──────┐
│              │      │ (e.g., comparator)│      │              │      │
└──────────────┘      └──────────────────┘      └──────────────┘      │
                                                                       │
    Radioactive                                                        │    ┌──────────────────────────┐
isotope generating                                                     │    │        Processor         │
  pair annihilation   ☆                                                ├────│ ⎛Determining coincidence⎞ │
    gamma rays                                                         │    │ ⎜     and producing      ⎟ │
 (e.g., 68Ge or 22Na.)                                                 │    │ ⎝    timing histogram    ⎠ │
                                                                       │    └──────────────────────────┘
┌──────────────┐      ┌──────────────────┐      ┌──────────────┐      │
│  Reference   │      │  Time pick-off   │      │              │      │
│   detector   │──────│     module       │──────│     TDC      │──────┘
│              │      │ (e.g., comparator)│      │              │
└──────────────┘      └──────────────────┘      └──────────────┘
```

F I G. 10

**EP 2 910 975 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101856236
- WO 2008102422 A
- US 4309611 A